# EUROPÄISCHE PATENTSCHRIFT

(11) **EP 2 571 844 B1**
(45) Veröffentlichungstag und Bekanntmachung des Hinweises auf die Patenterteilung: **04.10.2017**
(21) Anmeldenummer: 11718109.9
(22) Anmeldetag: 09.05.2011
(51) Int. Cl.: C07C 209/36, C07C 211/49

(54) **VERFAHREN ZUR HERSTELLUNG VON TOLUYLENDIAMIN DURCH HYDRIERUNG VON DINITROTOLUOL**
PROCESS FOR THE PREPARATION OF TULUENEDIAMINE BY HYDROGENATION OF DINITROTOLUENE
PROCÉDÉ DE PRODUCTION DE TOLUÈNE DIAMINE PAR HYDROGÉNATION DE DINITROTOLUÈNE

(30) Priorität: 17.05.2010 EP 10162924
(43) Veröffentlichungstag der Anmeldung: 27.03.2013
(73) Patentinhaber: BASF SE, 67056 Ludwigshafen am Rhein (DE)
(72) Erfinder: RAICHLE, Andreas, 01109 Dresden (DE); COELHO TSOU, Joana, 1190 Bruxelles (BE); NETO, Samuel, 1050 Bruxelles (BE); PENZEL, Urlich, 01945 Tettau (DE); OEHLENSCHLÄGER, Steffen, 2050 Antwerpen (BE); ZÖLLINGER, Michael, 73054 Eislingen (DE); BRAUNSBERG, Holgar, 01968 Senftenberg (DE); HAASE, Stefanie, 01900 Bretnig-Hauswalde (DE); BÜTTNER, Johannes, 01945 Ruhland (DE)
(86) Internationale Anmeldenummer: PCT/EP2011/057427
(87) Internationale Veröffentlichungsnummer: WO 2011/144481

(56) Entgegenhaltungen:
- WO-A1-00/35852
- WO-A1-2008/138784

## Beschreibung

Die Erfindung betrifft ein Verfahren zur Herstellung von Toluylendiamin durch Hydrierung von Dinitrotoluol.

Toluylendiamin, im Folgenden als TDA bezeichnet, ist ein in der Technik häufig eingesetztes aromatisches Amin; es wird insbesondere zu Toluylendiisocyanat weiterverarbeitet, das überwiegend in der Polyurethan-Herstellung Verwendung findet. TDA wird großtechnisch durch katalytische Hydrierung von Dinitrotoluol, im Folgenden als DNT bezeichnet, hergestellt.

Es wurden eine Vielzahl von Katalysatoren für die obige Umsetzung entwickelt, um eine möglichst hohe Ausbeute und Selektivität in der Umsetzung zu erreichen und darüber hinaus Katalysatoren aufzufinden, die auch bei höheren Reaktionstemperaturen stabil sind.

Die Hydrierung von DNT ist stark exotherm. Somit war es schon immer das Bestreben, die Reaktionswärme zum Beispiel in Form von Dampf zu nutzen.

Als ein für Abführung der Reaktionswärme besonders geeigneter Reaktor wurde in der WO 00/35852 A1 ein Reaktor mit interner und externer Schlaufenbewegung vorgeschlagen, der als vertikal aufrecht stehender Apparat ausgebildet ist, mit einer Treibstahldüse an seinem oberen Ende, über die in den oberen Bereich des Reaktors das aus dem Reaktionssumpf abgezogene Reaktionsgemisch über eine exotherme Schleife eingedüst wird, und das anschließend in ein zentrales Einsteckrohr, welches in Reaktorlängsrichtung angeordnet ist, einströmt, dieses von oben nach unten durchströmt, dort an einer Prallplatte umgelenkt wird und in einer internen Schlaufenbewegung außerhalb des Einsteckrohres erneut nach oben strömt. Für die Abführung der Reaktionswärme sind im Reaktorinnenraum Wärmetauscher, insbesondere Fieldrohre vorgesehen, d.h. vertikal in Reaktorlängsrichtung angeordnete Doppelrohre, wovon das innere Rohr unten zum äußeren Rohr hin offen und das äußere Rohr unten zum Reaktionsraum hin geschlossen ist und in denen ein Wärmeträger, insbesondere Wasser, strömt und die Reaktionswärme abführt. Zusätzlich zur Wärmeabführung über im Reaktorinnenraum angeordnete Wärmetauscher kann auch ein Wärmetauscher in der externen Schlaufenströmung vorgesehen sein. Bei dem in WO 00/35852 A1 beschriebenen Verfahren sollen aromatische Amine mit einer hohen Raum-Zeit-Ausbeute und bei deutlicher Unterdrückung von Nebenreaktionen hergestellt werden.

Es hat sich allerdings gezeigt, dass im Reaktor, insbesondere am Ort der DNT-Dosierung und in der nach unten gerichteten Strömung, mitunter erhöhte Gehalte an Nitroaromaten auftreten, insbesondere bei Verwendung geringerer Katalysatorgehalte.

In DE 198 44 901 C1 wird ein Verfahren zur Herstellung von aromatischen Aminen nach dem Sumpfphasenverfahren beschrieben, bei dem die Nitroaromaten über eine Ringleitung mit Löchern in den Reaktor eingespeist werden. Die Ringleitung kann auch über einen außenliegenden Wärmeträger gekühlt werden, um die Gefahr einer Überhitzung und damit einer thermischen Zersetzung der Nitroaromaten auszuschließen. Bei diesem Verfahren soll eine besonders gute Verteilung der Nitroaromaten in der Reaktionsmischung erreicht werden. Als geeignete Reaktoren werden beispielsweise Loop-Reaktoren, Blasensäulen, vorzugsweise Rührkessel, beschrieben. Die Vorteile des in der DE 198 44 901 C1 beschriebenen Verfahrens sollen darin liegen, dass sowohl die Katalysatordesaktivierung als auch die Nebenproduktbildung deutlich vermindert ist.
Die Herausforderung bei den obigen Verfahren zur Herstellung von TDA ist es, Verfahren zur Verfügung zu stellen, im Rahmen derer die Katalysatordesaktivierung und die Nebenproduktbildung vermindert wird. Es war daher die Aufgabe der vorliegenden Erfindung, ein wirtschaftlich attraktives Verfahren zur katalytischen Hydrierung von DNT zu TDA zur Verfügung zu stellen, das eine erhöhte Katalysatorstandzeit und Selektivität der Umsetzung von DNT zu TDA gewährleistet.
Gegenstand der vorliegenden Erfindung ist somit ein Verfahren zur kontinuierlichen Herstellung von Toluylendiamin durch Flüssigphasenhydrierung von Dinitrotoluol mit Wasserstoff in Gegenwart eines suspendierten, Nickel enthaltenden Katalysators, in einem Reaktor, mit einer dem Reaktor nachgeschalteten Produktabtrenneinheit, unter Erhalt eines Produktaustrages aus dem Reaktor umfassend eine Flüssigphase, enthaltend Toluylendiamin und Dinitrotoluol, in der der Nickel enthaltende Katalysator suspendiert ist, das dadurch gekennzeichnet ist, dass die Konzentration an Dinitrotoluol in der Flüssigphase des Produktaustrages aus dem Reaktor im Bereich zwischen dem Reaktor und der nachgeschalteten Produktabtrenneinheit auf einen Wert im Bereich von 1 bis 200 Gew.-ppm, bezogen auf das Gesamtgewicht der Flüssigphase des Produktaustrages aus dem Reaktor, eingestellt wird, wobei die Einstellung der Dinitrotoluol-Konzentration im flüssigen Produktaustrag aus dem Reaktor im Bereich zwischen dem Reaktor und der nachgeschalteten Produktabtrenneinheit auf der Basis einer wiederholten Messung der Dinitrotoluol-Konzentration im flüssigen Produktaustrag aus dem Reaktor in einem zeitlichen Abstand von ≤ 24 h durchgeführt wird und wobei die Nebenproduktbildung verringert und die Katalysatordesaktivierung weitgehend vermieden wird. Es wurde gefunden, dass durch die Sicherstellung einer DNT-Mindestkonzentration von mindestens 1 ppm, beispielsweise durch Absenkung der Reaktionstemperatur, des Wasserstoff-Partialdrucks, der Verweilzeit, der Katalysatorkonzentration oder Katalysatoraktivität bzw. durch Erhöhung der zugeführten Dinitrotoluolmenge, die Nebenproduktbildung erheblich verringert werden kann.

Weiterhin wurde gefunden, dass durch die Begrenzung der DNT-Konzentration auf maximal 200 ppm, Katalysatordesaktivierung weitgehend vermieden werden kann.

Zusammenfassend wurde damit gefunden, dass durch die Einstellung der Dinitrotoluol-Konzentration auf einen Wert im Bereich von 1 bis 200 ppm sowohl die Nebenproduktbildung verringert als auch die Katalysatordesaktivierung weitgehend vermieden werden kann.

Bevorzugt wird die Dinitrotoluol-Konzentration im flüssigen Produktaustrag aus dem Reaktor auf einen Wert im Bereich von 1 bis 100 Gew.-ppm, bezogen auf das Gesamtgewicht des flüssigen Produktaustrages aus dem Reaktor, weiter bevorzugt auf einen Wert von 2 bis 50 Gew.-ppm, bezogen auf das Gesamtgewicht des flüssigen Produktaustrages aus dem Reaktor und besonders bevorzugt auf einen Wert im Bereich von 3 bis 30 Gew.-ppm, bezogen auf das Gesamtgewicht des flüssigen Produktaustrages aus dem Reaktor, eingestellt.

"Flüssiger Produktaustrag im Bereich zwischen dem Reaktor und der Produktabtrenneinheit" bedeutet im Rahmen der Erfindung ein Flüssigkeitsstrom oder mehrere Flüssigkeitsströme, über den oder die der Produktaustrag aus dem Reaktor erfolgt. Bevorzugt erfolgt der Produktaustrag an einem Ort geringer DNT-Konzentration, also typischerweise an einer der DNT-Zudosierung abgewandten Stelle über eine Katalysatorabtrenneinheit. Bei Rührkesselreaktoren wird hierfür häufig ein einfacher Überlauf zur Katalysatorabtrenneinheit genutzt. Bei Schlaufenreaktoren erfolgt der Produktaustrag hingegen meist aus der äußeren Schlaufenströmung.

Die Sicherstellung einer DNT-Konzentration zwischen 1 und 200 ppm in genau diesem "flüssigen Produktaustrag im Bereich zwischen dem Reaktor und der Produktabtrenneinheit" dient damit gleichsam einer abschließenden Kontrolle der Misch- und Reaktionsprozesse in den vorgelagerten Reaktoren. Auf die technisch sehr viel aufwändigere Möglichkeit der Sicherstellung geeigneter DNT-Konzentrationen an verschiedenen Stellen direkt im Reaktor sei an dieser Stelle explizit hingewiesen. Die einzustellenden Konzentrationsbereiche würden sich in diesem Fall mit zunehmender Nähe zum Ort der DNT-Zudosierung (und/oder zu schlechter durchströmten Bereichen des Reaktors) erheblich erhöhen.

Die Produkt- bzw. Katalysatorabtrenneinheit ist allgemein ein Filter (z.B. ein Membranfilter / Querstromfilter), ein statischer Dekanter (z.B. ein Schwerkraftabscheider bzw. Settler, häufig ein Lamellenklärer) oder ein dynamischer Dekanter (z.B. eine Zentrifuge oder ein Düsenseparator). Der Katalysator wird von dem Produkt abgetrennt und anschließend (in der Regel als eingedickte Suspension) in den Reaktor zurückgeführt. Besonders bevorzugt erfolgt der Produktaustrag unter Rückhaltung des Katalysators. Das Amin kann danach nach den üblichen und bekannten Verfahren, beispielsweise durch Destillation oder Extraktion, gereinigt werden.

Die Einstellung der DNT-Konzentration im flüssigen Produktaustrag aus dem Reaktor im Bereich zwischen dem Reaktor und der nachgeschalteten Produktabtrenneinheit auf der Basis einer wiederholten Messung der Dinitrotoluol-Konzentration im flüssigen Produktaustrag aus dem Reaktor wird in einem zeitlichen Abstand von ≤ 24 h durchgeführt. Bevorzugt wird die Messung in einem zeitlichen Abstand von ≤ 12 h, besonders bevorzugt ≤ 4 h und ganz besonders bevorzugt ≤ 1 h fortgesetzt wiederholt. Die zeitlichen Abstände der Überwachung sind dabei so zu wählen, dass rasch auf Veränderungen der DNT-Konzentration im Bereich zwischen Reaktor und Produktabtrenneinheit reagiert werden kann. Die zur Überwachung zu entnehmenden Proben werden üblicherweise vor der Produktabtrenneinheit (beispielsweise bei Verwendung eines Settlers) oder nach der Produktabtrenneinheit (beispielsweise bei der Verwendung eines Filters) entnommen. Die Messung der DNT-Konzentration kann inline, online oder offline erfolgen.

Als Reaktoren, in denen das erfindungsgemäße Verfahren durchgeführt werden kann, kommen prinzipiell alle für die kontinuierliche Hydrierung von Nitroaromaten geeigneten Reaktoren in Frage. Geeignete Reaktoren für das erfindungsgemäße Verfahren sind kontinuierlich betriebene Rührkesselreaktoren, ebenso geeignet sind Kreisstrom- bzw. Loop-Reaktoren (Schlaufenreaktoren), sowie Blasensäulen. Bevorzugt werden für das erfindungsgemäße Verfahren Rührkesselreaktoren, Schlaufenreaktoren, Blasensäulen oder Strahlschlaufenreaktoren mit innerem und äußerem Kreislauf, wie beispielsweise in WO 00/35852 A1 beschrieben, genutzt.

In dem erfindungsgemäßen Verfahren wird DNT zu TDA hydriert. Dabei wird eine besonders geringe Nebenproduktbildung und Katalysatordesaktivierung beobachtet.

Die Hydrierung wird im erfindungsgemäßen Verfahren unter einem Druck im Bereich von 5 bis 50 bar, bevorzugt bei einem Druck im Bereich von 10 bis 40 bar, besonders bevorzugt bei einem Druck im Bereich von 20 bis 30 bar, durchgeführt. Die Betriebstemperatur, bei der die Hydrierung von Dinitrotoluol zu Toluylendiamin durchgeführt wird, liegt allgemein im Bereich von 50 bis 250 °C, bevorzugt im Bereich von 80 bis 200 °C, bevorzugt im Bereich von 105 bis 130 °.

Gemäß einer weiteren bevorzugten Ausführungsform der Erfindung wird das erfindungsgemäße Verfahren so betrieben, dass auch die Konzentration des teilhydrierten Zwischenproduktes Aminonitrotoluol, im Folgenden abgekürzt als ANT bezeichnet, kontrolliert wird, und zwar im Bereich zwischen den Reaktor und der Produktabtrenneinheit auf eine ANT-Konzentration im Bereich von 0 bis 2.000 ppm, bevorzugt auf eine ANT-Konzentration im Bereich von 0,5 bis 1.000 ppm, besonders bevorzugt eine ANT-Konzentration im Bereich von1 bis 200 ppm, eingestellt wird. Die Einstellung der Konzentration dieses teilhydrierten Zwischenprodukts in den genannten Bereichen ermöglicht zum einen eine weitere Reduzierung der Katalysatordesaktivierung und zum anderen eine weitere Reduzierung der Nebenproduktbildung. In der Regel ist der Verlauf der Konzentrationen von DNT und ANT weitgehend parallel.

Zur Herstellung von TDA kommen eine Vielzahl von Katalysatoren in Frage.

Gemäß einer ersten allgemeinen Ausführungsform der Erfindung wird das erfindungsgemäße Verfahren, wie oben ausgeführt, in Gegenwart eines Katalysators, insbesondere in Gegenwart eines Trägerkatalysators, der als Aktivkomponente Nickel allein oder zusammen mit mindestens einem Metall der I., V., VI. und/oder VIII. Nebengruppe des Periodensystems aufweist, durchgeführt. Die erfindungsgemäß verwendeten Katalysatoren können technisch durch Aufbringen von Nickel und gegebenenfalls mindestens einem der oben genannten zusätzlichen Metalle auf einen geeigneten Träger hergestellt werden.

Gemäß einer bevorzugten Ausführungsform der Erfindung weist der Katalysator einen Nickelgehalt zwischen 0,1 und 99 Gew.-%, bevorzugt zwischen 1 und 90 Gew.-%, besonders bevorzugt zwischen 25 und 85 Gew.-% und ganz besonders bevorzugt zwischen 60 und 80 Gew.-%, bezogen auf das Gesamtgewicht des Katalysators auf.

Vorzugsweise werden als Metalle der I., II., V., VI. und/oder VIII. Nebengruppe des Periodensystems Palladium, Platin, Rhodium, Eisen, Kobalt, Zink, Chrom, Vanadium, Kupfer, Silber oder ein Gemisch aus zwei oder mehreren davon verwendet.

Gemäß einer bevorzugten Ausführungsform der Erfindung enthält der Katalysator Ni und Platin. Gemäß einer weiteren bevorzugten Ausführungsform der Erfindung enthält der Katalysator Ni und Al; gemäß einer weiteren besonders bevorzugten Ausführungsform enthält der Katalysator Nickel, Palladium und Eisen.

Als Trägermaterialien werden vorzugsweise Aktivkohle, Ruß, Graphit, oder oxidische Trägerkomponenten wie Siliziumdioxid, Siliziumcarbid, Kieselgur, Aluminiumoxid, Magnesiumoxid, Titandioxid, Zirkoniumdioxid und/oder Hafniumdioxid oder ein Gemisch aus zwei oder mehr davon, besonders bevorzugt Zirkoniumdioxid, ZrO₂, HfO₂ und/oder SiO₂, ZrO₂ und/oder SiO₂, ZrO₂, HfO₂, verwendet.

Die verwendeten Träger sind vorzugsweise mesoporös und besitzen einen mittleren Porendurchmesser von 35 bis 50 nm und eine spezifische Oberfläche von 50 bis 250 m²/g. Die Oberfläche des Trägers wird bestimmt nach dem BET-Verfahren durch N₂-Adsorption, insbesondere nach DIN 66131. Die Bestimmung des mittleren Porendurchmessers und der Porengrößenverteilung erfolgt durch Hg-Porosimetrie, insbesondere nach DIN 66133.

Das Aufbringen von Nickel und gegebenenfalls des mindestens einen weiteren Metalls kann durch die üblichen geeigneten Verfahren, die dem Fachmann auf dem Gebiet der Katalysatortechnik bekannt sind, erreicht werden. Die mit dem Metall bzw. Metallsalz beschichteten, durch gemeinsame Fällung beschichteten oder getränkten Träger werden anschließend nach bekannten Verfahren getrocknet und kalziniert. Darauf folgend werden die beschichteten Träger durch Behandlung derselben in einem Gasstrom, der freien Wasserstoff enthält, aktiviert. Diese Aktivierung findet zumeist bei Temperaturen im Bereich von 30 bis 600 °C, vorzugsweise im Bereich von 80 bis 150 °C und insbesondere bevorzugt bei 100 °C statt. Der Gasstrom besteht vorzugsweise aus 50 bis 100 Vol.-% Wasserstoff und 0 bis 50 Vol.-% Stickstoff. Der für den erfindungsgemäßen Einsatz hergestellte Katalysator weist nach einstündiger Reduktion bei 100 °C einen Reduktionsgrad von mindestens 70 % auf.

Die so erhaltenen Trägerkatalysatoren besitzen im Allgemeinen eine Nickel-Metalloberfläche von ungefähr 10 bis ungefähr 50 m²/g, vorzugsweise ungefähr 20 bis ungefähr 60 m²/g. Der Nickelgehalt der im Rahmen des erfindungsgemäßen Verfahrens eingesetzten Katalysatoren liegt allgemein im Bereich von 0,1 bis 99 Gew.-%, bevorzugt im Bereich von 1 bis 90 Gew.-%, besonders bevorzugt im Bereich von 25 bis 85 Gew.-%, bezogen auf das Gesamtgewicht der eingesetzten Katalysatoren.

Geeignete Katalysatoren dieser Ausführungsform sind z.B. in den Druckschriften EP 1 161 297 A1 und EP 1 165 231 A1 beschrieben.

Gemäß einer zweiten Ausführungsform der Erfindung werden im Rahmen des erfindungsgemäßen Verfahrens aktivierte Nickelkatalysatoren, wie beispielsweise in der WO 2008/145179 A1 beschrieben, eingesetzt. Demgemäß werden gemäß einer bevorzugten Ausführungsform der Erfindung aktivierte Nickelkatalysatoren auf Basis einer Ni/Al-Legierung, die ein oder mehrere Metalle ausgewählt aus der Gruppe Mg, Ce, Ti, V, Nb, Cr, W, Mn, Re, Fe, Ru, Co, Rh, Ir, Pt, Cu, Ag, Au und Bi enthalten können, eingesetzt. Der Dotierungsgrad liegt dabei im Bereich von 0,05 Gew.-% bis 20 Gew.-% für jedes Dotierungselement. Die mittlere Partikelgröße der eingesetzten Katalysatoren ist < 25 µm.

Gemäß einer dritten Ausführungsform der Erfindung werden im Rahmen des erfindungsgemäßen Verfahrens Katalysatoren eingesetzt, die beispielsweise in der WO 2008/138784 A1 beschrieben sind. Gegenstand der Erfindung ist somit gemäß einer bevorzugten Ausführungsform der Erfindung weiterhin die Verwendung von Hydrierkatalysatoren, enthaltend als aktive Komponente ein Gemisch von Nickel, Palladium und einem zusätzlichen Element, ausgewählt aus der Gruppe, enthaltend Kobalt, Eisen, Vanadium, Mangan, Chrom, Platin, Iridium, Gold, Bismut, Molybdän, Selen, Tellur, Zinn und Antimon auf einem Träger, zur Herstellung von aromatischen Aminen durch katalytische Hydrierung der entsprechenden Nitroverbindungen, insbesondere zur Herstellung von Toluylendiamin durch Hydrierung von Dinitrotoluol. Das zusätzliche Element ist vorzugsweise ausgewählt aus der Gruppe, enthaltend Kobalt, Eisen, Vanadium, Bismut und Zinn.

Als Träger für die Katalysatoren können die hierfür üblichen und bekannten Materialien eingesetzt werden. Vorzugsweise werden Aktivkohle, Ruß, Graphit oder Metalloxide, bevorzugt hydrothermal stabile Metalloxide wie z.B. ZrO₂, TiO₂, Al₂O₃ eingesetzt. Bei Graphit sind die HSAG (High Surface Area Graphite) mit einer Oberfläche von 50 bis 300 m²/g besonders bevorzugt. Besonders bevorzugt sind Aktivkohlen, insbesondere physikalisch oder chemisch aktivierte Aktivkohlen, oder Ruße, wie Acetylenruß

Die Herstellung der erfindungsgemäßen Katalysatoren erfolgt beispielsweise, indem der Träger vorgelegt und mit einer wässrigen Lösung der Palladium- und Nickelsalze zusammen mit dem Zusatzelement gebracht wird. Dabei sollte die Menge des zur Lösung der Salze verwendeten Wassers so bemessen sein, dass eine knetbare Paste entsteht. Vorzugsweise wird das Wasser in einer Menge von 100 bis 200 Gew.-% der Trägermasse eingesetzt. Als Metallsalze kommen insbesondere Nitrate oder Chloride zum Einsatz, wobei Nitrate wegen ihrer geringeren Korrosivität bevorzugt sind. Die Paste wird gemischt und danach das Wasser bei geringem Druck und Temperaturen im Bereich zwischen 50 und 100 °C verdampft, beispielsweise in einem Rotationsverdampfer oder einem Ofen. Aus Sicherheitsgründen kann das Verdampfen in einem Stickstoffstrom erfolgen. Die Fixierung der Metalle auf dem Träger kann bei der Verwendung von Chloriden als Metallsalze durch Reduktion mit Wasserstoff erfolgen. Hierbei kann jedoch Korrosion auftreten. Bevorzugt werden die Metalle daher alkalisch fixiert. Dies erfolgt insbesondere durch Zugabe einer wässrigen Lösung von Alkalicarbonaten und nachfolgendes anionfrei waschen des Trägers. Alternativ können die Metalle auch alkalisch, insbesondere bei einem pH-Wert im Bereich von 8 bis 9, aus einer überstehenden Lösung auf den Träger gefällt werden. Danach wird der Träger getrocknet, vorzugsweise wie oben beschrieben, und mit Wasserstoff reduziert. Dies kann beispielsweise in einem Drehkugelofen geschehen. Vor dem Ausbau des Katalysators wird dieser passiviert, beispielsweise unter einem Inertgas, wie Stickstoff, der Spuren von Luft, vorzugsweise nicht mehr als 10 Vol.-%, enthält.

Die nach diesem Verfahren hergestellten erfindungsgemäßen Hydrierkatalysatoren enthalten vorzugsweise 0,5 bis 5 Gew.-% Palladium, 10 bis 20 Gew.-% Nickel und 0,5 bis 5 Gew.-% des Zusatzelementes.

Gemäß einer weiteren Ausführungsform der Herstellung der erfindungsgemäß eingesetzten Hydrierkatalysatoren erfolgt die Reduktion der Katalysatoren durch Zusatz von reduzierend wirkenden Salzen, wie Ammoniumcarboxylaten oder Alkalicarboxylaten, beispielsweise Ammoniumformiat oder Natriumformiat. Dazu wird der Träger mit Wasser suspendiert und gleichzeitig oder nach der Suspendierung die Lösungen der Metallsalze zugegeben. Als Metallsalze kommen insbesondere Nitrate oder Chloride zum Einsatz, wobei Nitrate wegen ihrer geringeren Korrosivität bevorzugt sind. Zu dieser Lösung werden die reduzierend wirkenden Salze zugegeben und die Suspension erhitzt, beispielsweise durch Kochen unter Rückfluss. Anschließend werden die Katalysatoren anionenfrei gewaschen und filtriert, beispielsweise durch eine Filterpresse oder eine Zentrifuge, und als feuchte Paste verwendet. Weitere Beispiele für die Herstellung der bevorzugt eingesetzten Katalysatoren finden sich in der WO 2005/037768 A1.

Im Rahmen des erfindungsgemäßen Verfahrens werden 2,4-DNT oder dessen technische Gemische weiterhin enthaltend 2,6-DNT, wobei diese Gemische vorzugsweise bis zu 35 Gew.-%, bezogen auf das Gesamtgemisch, an 2,6-DNT mit Anteilen von 1 bis 4 % an vicinalem DNT und 0,5 bis 1,5 % an 2,5- und 3,5-DNT aufweisen, zu dem entsprechenden Amin hydriert. Häufig werden die DNT-Isomeren im bei der zweifachen Nitrierung von Toluol anfallenden Isomerenverhältnis eingesetzt.

In dem erfindungsgemäßen Verfahren kann das 2,4-DNT oder das 2,4-DNT/2,6-DNT-Gemisch in reiner Form, als Mischung mit Wasser, als Mischung mit Wasser und einem alkoholischen Lösungsmittel oder als Mischung mit Wasser, einem alkoholischen Lösungsmittel und einem Katalysator reaktivierenden Zusatz eingesetzt werden. Ebenso können Katalysator, Wasser und/oder alkoholische Lösungsmittel oder Gemische hiervon gemeinsam oder getrennt zusätzlich zum DNT separat dosiert werden.

Wie sich aus dem oben Gesagten ergibt, kann beim erfindungsgemäßen Verfahren die Hydrierung unter Abwesenheit oder in Gegenwart eines alkoholischen Lösungsmittels und eines Katalysator aktivierenden Zusatzes durchgeführt werden.

Sofern ein alkoholisches Lösungsmittel und ein Katalysator reaktivierender Zusatz verwendet werden, können selbstverständlich auch Gemische aus zwei oder mehr davon zugesetzt werden.

Als alkoholische Lösungsmittel werden niedere aliphatische Alkohole mit 1 bis 6 Kohlenstoffatomen, vorzugsweise Methanol, Ethanol oder Propanol einzeln oder ein Gemisch aus zwei oder mehreren davon verwendet.

Als Katalysator aktivierende Zusätze werden vorzugsweise aprotische Lösungsmittel, insbesondere Aceton, Dimethylformamid, Dioxan oder Tetrahydrofuran oder ein Gemisch aus zwei oder mehreren davon eingesetzt.

Die Menge der eingesetzten alkoholischen Lösungsmittel und der Katalysator reaktivierenden Zusätze ist im Rahmen des erfindungsgemäßen Verfahrens nicht in besonderer Weise beschränkt und kann je nach Bedarf vom Fachmann frei gewählt werden.

Gemäß einer bevorzugten Ausführungsform der Erfindung wird die Hydrierung in einem Dreiphasengemisch aus wasserstoffhaltiger Gasphase, suspendiertem Katalysator und Flüssigphase enthaltend 0 bis 40 Vol-% eines Alkohols, 10 bis 60 Vol.-% Wasser und 20 bis 70 Vol-% TDA, wie vorstehend definiert, durchgeführt. Der Katalysatorgehalt beträgt dabei etwa 0,1 bis 15 Gew.-%, bevorzugt 2 bis 8 Gew.-%, bezogen auf das Gesamtgewicht des eingesetzten Dreiphasengemisches.

Die Erfindung wird anhand nachfolgend beschriebener Beispiele näher charakterisiert.

### Beispiele

### Beispiel 1

Es wurde ein zylinderförmiger Schlaufenreaktor mit einem durch zwei in Reihe geschalteten Kreiselpumpen angetriebenen, in einer zentral am Reaktorkopf angeordneten Treibstrahldüse mündenden externen Kreislauf, einem konzentrischen Einsteckrohr sowie einer Prallplatte im unteren Reaktorteil zur Umlenkung der Schlaufenströmung (interner Kreislauf) eingesetzt (zum Funktionsprinzip vgl. WO2000/35852 A1). Das Reaktionsvolumen des Reaktors betrug rund 14 m³. Der Reaktor war zur Abfuhr der Reaktionswärme mit einem Rohrbündel aus parallel geschalteten Fieldrohren versehen. Die Menge des in die Fieldrohre eingespeisten Kühlwassers wurde so eingestellt, dass die Temperatur im Reaktor bei ca. 120 °C gehalten wurde. Zur Aufrechterhaltung der Schlaufenströmung wurde im externen Produktkreislauf ein Volumenstrom von 600 m³/h umgewälzt, wodurch sich über die Treibstrahldüse ein Druckverlust von ca. 2,5 bar einstellte. Der Reaktor enthielt ca. 12 m³ eines flüssigen Hydrierbads. Dieses bestand im Wesentlichen aus einer Mischung von TDA und Wasser im Massenverhältnis von 0,58 : 0,42, in welcher ca. 5 Gew.-% eines auf SiO₂ und ZrO₂ geträgerten metallischen Ni-Katalysators (hergestellt nach Beispiel 2 der EP 1 161 297 und mittels einer Rührwerksmühle zerkleinert; hierbei bestanden 10 Vol-% des Katalysators aus Partikeln mit einem Durchmesseer ≤ ca. 5 µm, 50 Vol-% sind ≤ ca. 10 µm, und 90 Vol-% ≤ ca. 15 µm, gemessen mittels Laserbeugung (Malvern Mastersizer S) nach Aufrühren in Wasser) suspendiert und außerdem Wasserstoff gelöst waren. Der Flüssigkeitsspiegel befand sich hierbei knapp unterhalb der Mündung der Treibstrahldüse. Darüber befanden sich ca. 2 m³ einer Gasatmosphäre, deren Wasserstoffgehalt durch kontinuierliche Ausschleusung eines geringen Abgasstroms auf 90 bis 95 Vol-% (neben Inertgasen wie zum Beispiel N₂) eingestellt wurde. Mit einer Membrankolbendosierpumpe wurden in den Gasraum des Reaktors 7,5 t/h geschmolzenes und auf ca. 80 °C temperiertes DNT, bestehend aus einer Mischung der 2,4- und 2,6-DNT-Isomeren im Verhältnis von ca. 80 : 20, sowie ca. 5 % der übrigen DNT-Isomeren und Spuren von Mononitrotoluol eingedüst. Durch gleichzeitiges Einleiten von ca. 0,5 t/h Wasserstoff (verdünnt mit ca. 2 kg/h N₂) wurde im Reaktor ein Druck von 25 bar eingestellt. 95 % des Wasserstoffs wurden über einen Düsenring oberhalb der Prallplatte und 5 % am Reaktorausgang jeweils ins Hydrierbad dosiert. Die Reaktion verlief unter weitgehend isothermen Bedingungen: Im gesamten Reaktor bewegte sich die Reaktionstemperatur konstant zwischen 116 und 126 °C. Zusätzlich wurden über eine Membranpumpe ebenfalls kontinuierlich 625 kg/h einer Suspension des oben genannten Katalysators in Wasser (teilweise im Aufarbeitungsteil aus dem Hydrierprodukt abgetrennt) dosiert. Die Menge des enthaltenen Katalysators in dieser Suspension wurde hierbei zur Einstellung der DNT-Konzentration gezielt zwischen 0 und 5 kg/h variiert und betrug im Mittel etwa 1 kg/h.

Um den Flüssigkeitsstand im Reaktor konstant zu halten, wurde dem externen Produktkreislauf auf der Druckseite der 2. Kreiselpumpe kontinuierlich eine entsprechende Menge an Hydrierprodukt entnommen und in einen Lamellenklärer mit ca. 50 m³ Flüssigkeits- und ca. 10 m³ Gasvolumen geleitet. In dessen unterem Bereich konnte sich der Katalysator aufkonzentrieren. 18 Nm³/h einer entsprechend eingedickten Suspension wurden dann auf die Saugseite der 1. Kreiselpumpe zurückgeführt. Gleichzeitig wurden dem Lamellenklärer über einen Überlauf ca. 8,6 t/h Hydrierprodukt entnommen. Dieses enthielt ca. 4,9 t/h TDA (mit einer Isomerenverteilung entsprechend der des eingesetzten DNT), ca. 0,1 t/h Leicht- und Schwersieder (im Verhältnis von etwa 20 : 80) sowie ca. 3,6 t/h Wasser und bis zu etwa 1 kg/h Katalysator (v.a. Feinanteil). Das Hydrierprodukt gelangte über eine Druckreduzierung ebenso wie die Hydrierprodukte anderer Reaktoren in einen gemeinsamen Zwischenbehälter und wurde von diesem kontinuierlich der destillativen Aufarbeitung zugeführt. Die produktberührenden Teile waren teilweise aus Schwarzstahl (i.d.R. St37) und teilweise aus Edelstahl (1.4571) gefertigt.

Zur Bestimmung des Gehalts an DNT und ANT im Hydrierbad wurden im Abstand von maximal 4 h aus der Leitung vom externen Produktkreislauf des Schlaufenreaktors zum Lamellenklärer Suspensionsproben entnommen. Diese wurden durch Filtration vom suspendierten Feststoff befreit und mittels Polarographie die Konzentration der enthaltenen Nitroverbindungen bestimmt. Durch Anpassung der Katalysatorkonzentration in der dem Reaktor zugeführten wässrigen Suspension (s.o.) wurde die DNT-Konzentration zwischen 3 und 30 ppm (im Mittel: 10 ppm) und die ANT-Konzentration zwischen 1 und 200 ppm (im Mittel: 3 ppm) eingestellt.

Der Reaktor wurde unter den genannten Bedingungen über 3 Monate hinweg ohne nennenswerte Unterbrechungen betrieben. In dieser Zeit nahm die mittels Röntgenpulverdiffraktometrie bestimmte Kristallitgröße des jeweils von den obigen Suspensionsproben abfiltrierten Ni-Katalysators durch Sinterung von 9 nm auf 14 nm zu. Innerhalb weiterer drei Monate unter diesen Bedingungen nahm die Kristallitgröße weiter auf dann 15 nm zu. Eine nennenswerte Veränderung der stöchiometrischen Zusammensetzung des Katalysators oder seiner katalytischen Performance wurde während der gesamten 6 Monate eben so wenig beobachtet wie eine Bildung von oxidierten Ni-Spezies (zum Beispiel Bildung von Ni(OH)₂ durch oxidative Vergiftung).

### Beispiel 2

Es wurde ein Rührkesselreaktor (Durchmesser: 2,8 m, Höhe: 4,7 m, Volumen: 23 m³, Material: St37) mit innen im Bereich des Reaktormantels filigran befestigten Kühlschlangen und einem doppelten Schrägblattrührer verwendet: Der größere obere Schrägblattkranz drückte das Hydrierbad im Reaktorinneren nach unten, welches dann entlang der Kühlschlangen wieder nach oben strömte; der untere Schrägblattkranz hingegen saugte die von dem hier ebenfalls verwendeten Lamellenklärer zurückfließende eingedickte Suspension an und drückte sie nach oben in die durch den oberen Kranz erzeugte Strömung. Die Menge des in die Kühlschlangen eingespeisten Kühlwassers wurde so eingestellt, dass die Temperatur im Reaktor zwischen 116 und 126 °C gehalten wurde. Der Reaktor enthielt ca. 18 m³ eines flüssigen Hydrierbads. Dieses bestand im Wesentlichen aus einer Mischung von TDA und Wasser im Massenverhältnis von 0,58 : 0,42, in welcher ca. 5 Gew.-% des in Beispiel 1 genannten, auf SiO₂ und ZrO₂ geträgerten metallischen Ni-Katalysators suspendiert und außerdem Wasserstoff gelöst waren. Oberhalb des Flüssigkeitsspiegels befanden sich ca. 5 m³ einer Gasatmosphäre, deren Wasserstoffgehalt durch kontinuierliche Ausschleusung eines geringen Abgasstroms auf 90 bis 99 Vol-% (neben Inertgasen wie z.B. N₂) eingestellt wurde.

Mit einer Membrankolbendosierpumpe wurden 5 t/h geschmolzenes und auf ca. 80 °C temperiertes DNT, bestehend aus einer Mischung der 2,4- und 2,6-DNT-lsomeren im Verhältnis von ca. 80 : 20, sowie ca. 5 % der übrigen DNT-Isomeren und Spuren von Mononitrotoluol in einen zum Gasraum hin offenen Trichter dosiert, welcher das DNT über eine Leitung nach unten und zwischen den Schrägblattkränzen ins Hydrierbad leitete. Durch gleichzeitiges Einleiten von ca. 330 kg/h Wasserstoff (verdünnt mit ca. 2 kg/h N₂) wurde im Reaktor ein Druck von 25 bar eingestellt. Der Wasserstoff wurde über einen zwischen den beiden Schrägblattrührerkränzen zentrisch angebrachten und dort filigran befestigten Düsenring ins Hydrierbad dosiert. Die Reaktion verlief unter weitgehend isothermen Bedingungen. Zusätzlich wurden über eine Membranpumpe ebenfalls kontinuierlich 435 kg/h einer Suspension des oben genannten Katalysators in Wasser (teilweise im Aufarbeitungsteil aus dem Hydrierprodukt abgetrennt) dosiert. Die Menge des enthaltenen Katalysators in dieser Suspension wurde hierbei zur Einstellung der DNT-Konzentration gezielt zwischen 0 und 5 kg/h variiert und betrug im Mittel knapp 1 kg/h.

Um den Flüssigkeitsstand im Reaktor konstant zu halten, wurde über einen Überlauf kontinuierlich eine entsprechende Menge an Hydrierprodukt entnommen und in einen Lamellenklärer mit ca. 16 m³ Flüssigkeits- und ca. 4 m³ Gasvolumen geleitet. In dessen unterem Bereich konnte sich der Katalysator aufkonzentrieren. Ca. 30 Nm³/h einer entsprechend eingedickten Suspension wurden dann mit Hilfe des unteren Kranzes des Schrägblattrührers in den Rührkessel zurückgesaugt. Gleichzeitig wurden dem Lamellenklären über einen Überlauf ca. 5,8 t/h Hydrierprodukt entnommen. Dieses enthielt ca. 3,3 t/h TDA (mit einer Isomerenverteilung entsprechend der des eingesetzten DNT), ca. 0,07 t/h Leicht- und Schwersieder (im Verhältnis von etwa 10 : 60) sowie ca. 2,4 t/h Wasser und bis zu 1 kg/h Katalysator (v.a. Feinanteil). Das Hydrierprodukt gelangte über eine Druckreduzierung ebenso wie die Hydrierprodukte anderer Reaktoren in einen gemeinsamen Zwischenbehälter und wurde von diesem kontinuierlich der destillativen Aufarbeitung zugeführt.

Zur Bestimmung des Gehalts an DNT und ANT im Hydrierbad wurden im Abstand von maximal 4 h aus der Leitung vom Rührkesselreaktor zum Lamellenklärer Suspensionsproben entnommen. Diese wurden durch Filtration vom suspendierten Feststoff befreit und mittels Polarographie die Konzentration der enthaltenen Nitroverbindungen bestimmt. Durch Anpassung der Katalysatorkonzentration in der dem Reaktor zugeführten wässrigen Suspension (s.o.) wurde die DNT-Konzentration zwischen 3 und 30 ppm (im Mittel: 10 ppm) und die Aminonitrotoulol-Konzentration zwischen 1 und 200 ppm (im Mittel: 3 ppm) eingestellt.

Der Reaktor wurde unter den genannten Bedingungen über drei Monate hinweg ohne nennenswerte Unterbrechungen betrieben. In dieser Zeit nahm die mittels Röntgenpulverdiffraktometrie bestimmte Kristallitgröße des jeweils von den obigen Suspensionsproben abfiltrierten Ni-Katalysators durch Sinterung von 15 nm auf 16 nm zu. Eine nennenswerte Veränderung seiner stöchiometrischen Zusammensetzung oder seiner katalytischen Performance wurde ebenso wenig beobachtet wie eine Bildung von oxidierten Ni-Spezies (zum Beispiel Bildung von Ni(OH)₂ durch oxidative Vergiftung).

### Beispiel 3

Es wurde der im Beispiel 2 beschriebene Rührkesselreaktor verwendet und wie dort beschrieben verfahren. Um eine bessere Ablösung der Reaktionsprodukte vom Katalysator, eine Erschwerung der oxidativen Vergiftung und eine optimale Homogenität des Hydrierbads bereits bei einer tieferen Reaktionstemperatur von 100 bis 105 °C zu erreichen, wurde Ethanol als zusätzlicher Lösungsvermittler eingesetzt. Der Katalysator wurde entsprechend nicht in wässriger Suspension, sondern in Form von 0,5 t/h einer ethanolischen Suspension dosiert (großteils wurde hierbei im Aufarbeitungsteil aus dem Hydrierprodukt wiedergewonnenes Ethanol eingesetzt). Die Menge des enthaltenen Katalysators in dieser Suspension wurde wie im Beispiel 2 zur Einstellung der DNT-Konzentration gezielt zwischen 0 und 5 kg/h variiert und betrug im Mittel knapp 1 kg/h. Die übrigen Mengenströme waren wie in Beispiel 2 beschrieben.

Die dem Lamellenklärer über den Überlauf entnommenen ca. 5,9 t/h Hydrierprodukt enthielten gut 3,3 t/h TDA (mit einer Isomerenverteilung entsprechend der des eingesetzten DNT), ca. 0,05 t/h Leicht- und Schwersieder (im Verhältnis von etwa 10 : 40) sowie ca. 2 t/h Wasser, ca. 0,5 t/h Ethanol und bis zu 1 kg/h Katalysator (v.a. Feinanteil). Das Hydrierprodukt gelangte über eine Druckreduzierung ebenso wie die Hydrierprodukte anderer Reaktoren in einen gemeinsamen Zwischenbehälter und wurde von diesem kontinuierlich der destillativen Aufarbeitung zugeführt. Zur Bestimmung des Gehalts an DNT und ANT im Hydrierbad wurden im Abstand von maximal 4 h aus der Leitung vom Rührkesselreaktor zum Lamellenklärer Suspensionsproben entnommen. Diese wurden durch Filtration vom suspendierten Feststoff befreit und mittels Polarographie die Konzentration der enthaltenen Nitroverbindungen bestimmt. Durch Anpassung der Katalysatorkonzentration in der dem Reaktor zugeführten wässrigen Suspension (s.o.) wurde die DNT-Konzentration zwischen 3 und 30 ppm (im Mittel: 10 ppm) und die ANT-Konzentration zwischen 1 und 200 ppm (im Mittel: 3 ppm) eingestellt.

Der Reaktor wurde unter den genannten Bedingungen über drei Monate hinweg ohne nennenswerte Unterbrechungen betrieben. In dieser Zeit nahm die mittels Röntgenpulverdiffraktometrie bestimmte Kristallitgröße des jeweils von den obigen Suspensionsproben abfiltrierten Ni-Katalysators durch Sinterung von 14 nm auf 15 nm zu. Eine nennenswerte Veränderung seiner stöchiometrischen Zusammensetzung oder seiner katalytischen Performance wurde ebenso wenig beobachtet wie eine Bildung von oxidierten Ni-Spezies (z.B. Bildung von Ni(OH)₂ durch oxidative Vergiftung).

### Beispiel 4

Es wurde wie im Beispiel 2 ein Rührkesselreaktor (Durchmesser: 2,8 m, Höhe: 4,7 m, Volumen: 23 m³, Material: St37) mit innen im Bereich des Reaktormantels befestigten Kühlschlangen und einem doppelten Schrägblattrührer verwendet. Die Befestigungen der Kühlschlangen und des Düsenrings zur Wasserstoffverteilung waren hier allerdings sehr viel massiver ausgeführt und verursachten damit eine geringfügig schlechtere Durchmischung des Hydrierbads. Unabhängig hiervon wurde die Reaktion wie in Beispiel 2 beschrieben durchgeführt.

Trotz einer im Vergleich zu Beispiel 2 verdoppelten durchschnittlichen Konzentration des in der wässrigen Suspension zudosierten Frischkatalysators von 2 statt 1 kg/h - also einer doppelt so hohen Dosierung von Frischkatalysator - stieg die in den filtrierten, zwischen Reaktor und Lamellenklärer entnommenen Suspensionsproben bestimmte mittlere Aminonitrotoulol-Konzentration im Vergleich zum Beispiel 2 von 3 auf 25 ppm, vereinzelt wurden Werte bis zu 300 ppm gemessen. Auch die mittlere DNT-Konzentration war mit 15 statt 10 ppm leicht erhöht und vereinzelt wurden Werte bis zu 50 ppm gemessen.

Der Reaktor wurde unter den genannten Bedingungen über drei Monate hinweg ebenfalls ohne nennenswerte Unterbrechungen betrieben. In dieser Zeit nahm die mittels Röntgenpulverdiffraktometrie bestimmte Kristallitgröße des jeweils von den obigen Suspensionsproben abfiltrierten Ni-Katalysators durch Sinterung von 8 nm auf 17 nm sehr stark zu. Eine nennenswerte Veränderung seiner stöchiometrischen Zusammensetzung oder eine Bildung von oxidierten Ni-Spezies (z.B. Bildung von Ni(OH)₂ durch oxidative Vergiftung) trat auch hier nicht auf. Allerdings musste die DNT-Dosierung nach diesen drei Monaten schrittweise reduziert werden, um einen weiteren Anstieg der Konzentrationen von DNT und Aminonitrotoluol zu verhindern. Nach weiteren zwei Monaten war ein Austausch des gesamten Katalysators unvermeidlich.

### Vergleichsbeispiel 1

Es wurde der in Beispiel 4 beschriebene Rührkesselreaktor verwendet und wie dort beschrieben vorgegangen. Im Unterschied zu Beispiel 4 bestand das Hydrierbad hier allerdings aus einer Mischung von Toluylendiamin (TDA) und Wasser im Verhältnis von 0,3 : 0,7, welche neben dem gelösten Wasserstoff nur ca. 1,5 Gew.-% des in Beispiel 1 genannten auf SiO₂ und ZrO₂ geträgerten metallischen Ni-Katalysators suspendiert enthielt. Die Menge des in der zudosierten wässrigen Suspension enthaltenen Frischkatalysators betrug außerdem im Mittel nur knapp 0,5 kg/h.

Der Reaktor konnte unter diesen Bedingungen nur etwa eine Stunde betrieben werden. Dann musste die Reaktion aufgrund einer sinkenden Wasserstoffaufnahme abgebrochen werden. Die polarographische Bestimmung des Gehalts an DNT und Aminonitrotoluolen in der 30 min nach Reaktionsbeginn aus der Leitung vom Rührkesselreaktor zum Lamellenklärer entnommenen und filtrierten Suspensionsprobe ergab eine DNT-Konzentration von 842 ppm und eine Aminonitrotoulol-Konzentration von 2050 ppm. Eine anschließende Untersuchung des von einer nach Reaktionsabbruch entnommenen Suspensionsprobe abfiltrierten Ni-Katalysators ergab zwar im Rahmen der Messgenauigkeit eine unveränderte Ni-Kristallitgröße und stöchiometrische Zusammensetzung. Allerdings lag ein erheblicher Teil des Nickels in oxidierter Form (v.a. als katalytisch inaktives Ni(OH)₂) vor. Ein Austausch des gesamten Katalysators war unvermeidlich.

### Beispiel 5

Die Hydrierung von DNT zu TDA, wurde in einem 180 ml kontinuierlichen Rührkessel durchgeführt, der Katalysator wurde mechanisch im Reaktor zurückgehalten. Ein 2,8 % Pt-0,7 %Ni auf Norit® SX Aktivkohleträger Katalysator (WO2005/037768) wurde in Wasser suspendiert und in den Reaktor gebracht (Katalysatormenge 0,5 Gew.-% des Flüssigkeitsvolumens des Reaktors), dieser wurde auf eine Temperatur von 125°C temperiert. Unter einem Wasserstoffdruck von 24 bar wurde DNT in einer solchen Menge kontinuierlich als Schmelze zudosiert, dass eine Katalysatorbelastung von 180 kg_{DNT}/kg_{cat..}h eingestellt wurde. Proben wurden mittels Gaschromatographie analysiert: Die DNT- und ANT-Konzentration sowie die TDA-Ausbeute wurden verfolgt.

### Beispiel 6

Gleich wie Beispiel 5, aber mit Katalysatorbelastung von 229 kg_{DNT}/kg_{cat-}h.

### Vergleichsbeispiel 2

Gleich wie Beispiel 5, aber mit Katalysatorbelastung von 45 kg_{DNT}/kg_{cat}.h.

### Vergleichsbeispiel 3

Gleich wie Beispiel 5,aber mit Katalysatorbelastung von 284 kg_{DNT}/kg_{cat.}h.

| | TDA-Ausbeute (%) | DNT (ppm) | ANT (ppm) |
|---|---|---|---|
| Beispiel 5 | 99,1 | 29 | 600 |
| Beispiel 6 | 98,5 | 127 | 2450 |
| Vergleichsbeispiel 2 | 98,7 | 0 | 0 |
| Vergleichsbeispiel 3 | 96,4 | 1000 | 19500 |

### Beispiel 7

Die Hydrierung von DNT zu TDA, wurde in einem 180 ml kontinuierlichen Rührkessel durchgeführt, der Katalysator wurde mechanisch im Reaktor zurückgehalten. Ein Katalysator, wie in Beispiel 1 beschrieben, wurde in Wasser suspendiert und in den Reaktor gebracht (Katalysatormenge 2,5 Gew.-% des Flüssigkeitsvolumens des Reaktors), dieser wurde auf eine Temperatur von 125°C temperiert. Unter einem Wasserstoffdruck von 24 bar wurde DNT in einer solchen Menge kontinuierlich als Schmelze zudosiert, dass eine Katalysatorbelastung von 17 kg_{DNT}/kg_{cat.}h eingestellt wurde. Proben wurden mittels Gaschromatographie analysiert: Die DNT- und ANT-Konzentration sowie die TDA-Ausbeute wurden verfolgt.

### Vergleichsbeispiel 4

Gleich wie Beispiel 6, aber mit Katalysatorbelastung von 12 kg_{DNT}/kg_{cat.}h.

| | TDA-Ausbeute (%) | DNT (ppm) | ANT (ppm) |
|---|---|---|---|
| Beispiel 7 | 99,4 | 15 | 300 |
| Vergleichsbeispiel 4 | 98,3 | 0 | 0 |

### Vergleich unterschiedlicher Analysenmethoden zur Bestimmung von DNT und ANT:

Nachfolgend seien beispielhaft drei Verfahren zur Analytik von DNT und ANT im Hydrierbad aufgezeigt und verglichen. Dazu wurde eine Versuchsreihe angefertigt und die Analysenverfahren verglichen. Mit den aufgeführten Beispielen soll gezeigt werden, dass eine Untersuchung des Hydrierbades mit gängigen Analysenverfahren möglich ist und dass unabhängig von der Untersuchungsmethode dieselben Ergebnisse erhalten werden. Die drei Verfahren Gaschromatographie (GC), Hochdruckflüssigchromatographie (HPLC) und Polarographie stehen dabei nur beispielhaft für die Fülle der verschiedenen möglichen spektroskopischen, chromatographischen, elektrochemischen und anderen Methoden. Aus den nachfolgenden Ergebnissen wird ersichtlich, dass z.B. der Einsatz von Polarographie prinzipiell möglich ist, allerdings muss darauf geachtet werden, dass die Proben zeitnah (spätestens 4 h, besser 1 h, noch besser 30 min oder 15 min nach der Probenahme) vermessen werden. Ansonsten ergeben sich fälschlicherweise DNT- und ANT-Werte, die zu hoch sind. Mit GC und HPLC lassen sich die Substanzen identifizieren und quantifizieren, ohne das sich falsch positive Ergebnisse ergeben.

Die Analysemethoden werden bevorzugt wie folgt durchgeführt:
Die Polarographie erfolgt in einem Lösungsmittelgemisch, das aus gleichen Teilen Dimethylformamid und 1 Mol/l der Chlorsäurelösung besteht.

Dinitrotoluol (DNT) wird im angegebenen Elektrolyten bei ca. 330 mV und Aminonitrotoluol bei ca. 550 mV reduziert. Eine Veränderung des Isomerenverhältnisses beeinflusst die polarographische Stufenhöre nicht.
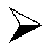 Polarograph VA-Processor 746 bzw. 693 mit VA-Stand (Fa. Metrohm)
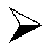 Quecksilbertropfelektrode, MME,
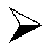 Bezugselektrode, Ag/AgCl,
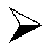 Elektrolytschlüssel, LiCl gesättigt in Ethanol,
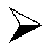 Meßkolben 100 ml, 50 ml und 25 ml,
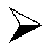 Analysenwaage (Ablesegenauigkeit 0,001g oder besser), z.B. Sartorius RC 250
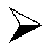 Pipette 5 ml,
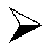 Titriergefässe

Die Gaschromatographie (GC) wird bevorzugt wie nachfolgend angegeben durchgeführt:

| | |
|---|---|
| Säule: | Varian - Factor Four CP 8977 (10m x 0,10mmID x 0,20µm FD) |
| Trägergas: | Helium |
| Pressure: | 41,8 psi |
| Split flow: | 15,2 ml/min |
| Temperatur (ECD-Detector) : | 300° C |
| Temperatur (Injektor): | 250°C |
| Ofen : Initialtemp.: | 75°C - 20.0°C/min→ 250°C - 4.0 min |
| Injektionsvolumen: | 0,2 µl |
| Probenvorbereitung: Einwaage (Probe): | ca. 500mg + 5ml MeOH |

Die Hochdruckflüssigkeits-Chromatographie (HPLC) wird bevorzugt wie folgt durchgeführt:

| | |
|---|---|
| Säule: | Agilent Lichrosorb RP-18 (4,6x 200 mm, 5µm) |
| Eluent 1: | Wasser+ 1g/l Ammoniumacetat |
| Eluent 2: | Methanol |
| Gradient: | 0 min 70% Wasser/ 30% Methanol |
| | 18 min 10% Wasser/ 90% Methanol |
| | 25 min 10% Wasser/ 90% Methanol |
| | 30 min 70% Wasser/ 30% Methanol |
| Fluß: | 0,5 ml/min |
| Ofentemp.: | 38 °C |
| Wellenlänge: | 254nm/ Bandbreite 4nm Referenz 550nm/ Bandbreite 100nm |
| Inj.volumen: | 25 µl |
| | |
| Probenvorbereitung: Einwaage (Probe): | ca. 500mg + 5ml MeOH |

### Versuchsreihe:

In einem 250 ml Erlenmeierkolben wurden Mischungen aus genau 60,00 g frisch destilliertem TDA (2,4- und 2,6-Isomer) und 40,00 g Wasser hergestellt und bei 85 °C gut vermischt. Sobald die Proben homogen waren, wurden unterschiedliche Mengen eines DNT-Isomerengemischs zugegeben und erneut homogenisiert. Dann wurde mittels GC und HPLC der Gehalt des 2,4-DNT-Isomers und mittels GC und Polarographie der Gesamtgehalt aller DNT-Isomeren bestimmt. Die Bestimmung mittels Polarographie wurde 15 min nach der erneuten Homogenisierung und ein zweites mal 4 h nach der erneuten Homogenisierung durchgeführt.

Ergebnisse (Messwerte in ppm):

| Probe | Methode | | 2,4-DNT | gesamt-DNT |
|---|---|---|---|---|
| 0 | GC | | 0 | 0 |
| | HPLC | | 0 | |
| | Polarographie | nach 15 min | | 0 |
| | | nach 4 h | | 45 |
| 1 | GC | | 18 | 20 |
| | HPLC | | 17 | |
| | Polarographie | nach 15 min | | 19 |
| | | nach 4 h | | 34 |
| 2 | GC | | 39 | 46 |
| | HPLC | | 37 | |
| | Polarographie | nach 15 min | | 49 |
| | | nach 4 h | | 69 |
| 3 | GC | | 55 | 65 |
| | HPLC | | 53 | |
| | Polarographie | nach 15 min | | 60 |
| | | nach 4 h | | 75 |

## Patentansprüche

1. Verfahren zur kontinuierlichen Herstellung von Toluylendiamin durch Flüssigphasenhydrierung von Dinitrotoluol mit Wasserstoff in Gegenwart eines suspendierten, Nickel enthaltenden Katalysators, in einem Reaktor, mit einer dem Reaktor nachgeschalteten Produktabtrenneinheit, unter Erhalt eines Produktaustrages aus dem Reaktor umfassend eine Flüssigphase, enthaltend Toluylendiamin und Dinitrotoluol, in der der Nickel enthaltende Katalysator suspendiert ist, **dadurch gekennzeichnet, dass** die Konzentration an Dinitrotoluol in der Flüssigphase des Produktaustrages aus dem Reaktor im Bereich zwischen dem Reaktor und der nachgeschalteten Produktabtrenneinheit auf einen Wert im Bereich von 1 bis 200 Gew.-ppm, bezogen auf das Gesamtgewicht der Flüssigphase des Produktaustrages aus dem Reaktor, eingestellt wird, wobei die Einstellung der Dinitrotoluol-Konzentration im flüssigen Produktaustrag aus dem Reaktor im Bereich zwischen dem Reaktor und der nachgeschalteten Produktabtrenneinheit auf der Basis einer wiederholten Messung der Dinitrotoluol-Konzentration im flüssigen Produktaustrag aus dem Reaktor in einem zeitlichen Abstand von ≤ 24 h durchgeführt wird und wobei die Nebenproduktbildung verringert und die Katalysatordesaktivierung weitgehend vermieden wird.

2. Verfahren nach Anspruch 1, **dadurch gekennzeichnet, dass** die Konzentration an Dinitrotoluol im flüssigen Produktaustrag aus dem Reaktor auf einen Wert im Bereich von 1 bis 100 Gew.-ppm, bezogen auf das Gesamtgewicht des flüssigen Produktaustrages aus dem Reaktor, bevorzugt auf einen Wert von 2 bis 50 Gew.-ppm, bezogen auf das Gesamtgewicht des flüssigen Produktaustrages aus dem Reaktor, weiter bevorzugt auf einen Wert im Bereich von 3 bis 30 Gew.-ppm, bezogen auf das Gesamtgewicht des flüssigen Produktaustrages aus dem Reaktor, eingestellt wird.

3. Verfahren nach Anspruch 1 oder 2, **dadurch gekennzeichnet, dass** die Hydrierung von Dinitrotoluol zu Toluylendiamin bei einer Temperatur im Bereich von 50 bis 250°C durchgeführt wird.

4. Verfahren nach einem der Ansprüche 1 bis 3, **dadurch gekennzeichnet, dass** die Hydrierung von Dinitrotoluol zu Toluylendiamin bei einem Druck im Bereich von 5 bis 50 bar durchgeführt wird.

5. Verfahren nach einem der Ansprüche 1 bis 4, **dadurch gekennzeichnet, dass** die Flüssigphase des Produktaustrages Aminonitrotoluol enthält und dass die Konzentration an Aminonitrotoluol in der Flüssigphase des Produktaustrages aus dem Reaktor im Bereich zwischen dem Reaktor und der Produktabtrenneinheit auf einen Wert im Bereich von 0 bis 2000 Gew.-ppm, bezogen auf das Gesamtgewicht der Flüssigphase des Produktaustrages, eingestellt wird.

6. Verfahren nach einem der Ansprüche 1 bis 5, **dadurch gekennzeichnet, dass** der Reaktor ausgewählt ist aus der Gruppe Rührkesselreaktor, Schlaufenreaktor und Blasensäule.

7. Verfahren nach einem der Ansprüche 1 bis 6, **dadurch gekennzeichnet, dass** der Reaktor ein Strahlschlaufenreaktor mit äußerem und innerem Kreislauf ist.

8. Verfahren nach einem der Ansprüche 1 bis 7, **dadurch gekennzeichnet, dass** die Produktabtrenneinheit ausgewählt ist aus der Gruppe Filter, statischer Dekanter und dynamischer Dekanter.

9. Verfahren nach einem der Ansprüche 1 bis 8, **dadurch gekennzeichnet, dass** der suspendierte Katalysator zusätzlich zu Nickel als katalytisch aktives Metall mindestens ein Metall der I., II., V., VI. und/oder VIII. Nebengruppe des Periodensystems enthält.

10. Verfahren nach einem der Ansprüche 1 bis 9, **dadurch gekennzeichnet, dass** der Katalysator einen Nickelgehalt zwischen 0,1 und 99 Gew.-%, bezogen auf das Gesamtgewicht des Katalysators, aufweist.

11. Verfahren nach einem der Ansprüche 1 bis 10, **dadurch gekennzeichnet, dass** der Katalysator mindestens eine oxidische Trägerkomponente enthält.

12. Verfahren nach einem der Ansprüche 1 bis 11, **dadurch gekennzeichnet, dass** der Katalysator Platin und Nickel enthält.

13. Verfahren nach einem der Ansprüche 1 bis 11, **dadurch gekennzeichnet, dass** der Katalysator Palladium, Nickel und Eisen enthält.

14. Verfahren nach einem der Ansprüche 1 bis 13, **dadurch gekennzeichnet, dass** der Katalysator in einer Menge von 0,1 bis 15 Gew.-%, bezogen auf das Gesamtgewicht der Reaktionsmischung im Reaktor, eingesetzt wird.

15. Verfahren nach einem der Ansprüche 1 bis 14, **dadurch gekennzeichnet, dass** die Reaktion in einem Dreiphasengemisch aus wasserstoffhaltiger Gasphase, suspendiertem Katalysator und Flüssigphase, enthaltend 0 bis 40 Vol.-% eines Alkohols, 10 bis 60 Vol.-% Wasser und 20 bis 70 Vol.-% Toluylendiamin, durchgeführt wird.

## Claims

1. A process for the continuous preparation of toluenediamine by liquid-phase hydrogenation of dinitrotoluene by means of hydrogen in the presence of a suspended, nickel-comprising catalyst in a reactor with a product isolation unit downstream of the reactor to give a product output from the reactor comprising a liquid phase comprising toluenediamine and dinitrotoluene, in which the nickel-comprising catalyst is suspended, wherein the concentration of dinitrotoluene in the liquid phase of the product output from the reactor in the region between the reactor and the downstream product isolation unit is set to a value in the range from 1 to 200 ppm by weight, based on the total weight of the liquid phase of the product output from the reactor, with the setting of the dinitrotoluene concentration in the liquid product output from the reactor in the region between the reactor and the downstream product isolation unit being carried out on the basis of a repeated measurement of the dinitrotoluene concentration in the liquid product output from the reactor at time intervals of ≤ 24 hours and with product formation being reduced and catalyst deactivation being largely avoided.

2. The process according to claim 1, wherein the concentration of dinitrotoluene in the liquid product output from the reactor is set to a value in the range from 1 to 100 ppm by weight, based on the total weight of the liquid product output from the reactor, preferably to a value of from 2 to 50 ppm by weight, based on the total weight of the liquid product output from the reactor, more preferably to a value in the range from 3 to 30 ppm by weight, based on the total weight of the liquid product output from the reactor.

3. The process according to claim 1 or 2, wherein the hydrogenation of dinitrotoluene to toluenediamine is carried out at a temperature in the range from 50 to 250°C.

4. The process according to any of claims 1 to 3, wherein the hydrogenation of dinitrotoluene to toluenediamine is carried out at a pressure in the range from 5 to 50 bar.

5. The process according to any of claims 1 to 4, wherein the liquid phase of the product output comprises aminonitrotoluene and the concentration of aminonitrotoluene in the liquid phase of the product output from the reactor in the region between the reactor and the product isolation unit is set to a value in the range from 0 to 2000 ppm by weight, based on the total weight of the liquid phase of the product output.

6. The process according to any of claims 1 to 5, wherein the reactor is selected from the group consisting of a stirred tank reactor, a loop reactor and a bubble column.

7. The process according to any of claims 1 to 6, wherein the reactor is a jet loop reactor having external and internal circulation.

8. The process according to any of claims 1 to 7, wherein the product isolation unit is selected from the group consisting of a filter, a static decanter and a dynamic decanter.

9. The process according to any of claims 1 to 8, wherein the suspended catalyst comprises at least one metal of transition groups I., II., V., VI. and/or VIII. of the Periodic Table in addition to nickel as catalytically active metal..

10. The process according to any of claims 1 to 9, wherein the catalyst has a nickel content in the range from 0.1 to 99% by weight, based on the total weight of the catalyst.

11. The process according to any of claims 1 to 10, wherein the catalyst comprises at least one oxidic support component.

12. The process according to any of claims 1 to 11, wherein the catalyst comprises platinum and nickel.

13. The process according to any of claims 1 to 11, wherein the catalyst comprises palladium, nickel and iron.

14. The process according to any of claims 1 to 13, wherein the catalyst is used in an amount of from 0.1 to 15% by weight, based on the total weight of the reaction mixture in the reactor.

15. The process according to any of claims 1 to 14, wherein the reaction is carried out in a three-phase mixture composed of hydrogen-comprising gas phase, suspended catalyst and liquid phase comprising from 0 to 40% by volume of an alcohol, from 10 to 60% by volume of water and from 20 to 70% by volume of toluenediamine.

## Revendications

1. Procédé de fabrication continue de toluylène-diamine par hydrogénation en phase liquide de dinitrotoluène avec de l'hydrogène en présence d'un catalyseur suspendu contenant du nickel, dans un réacteur, avec une unité de séparation de produits en aval du réacteur, pour obtenir une sortie de produits du réacteur comprenant une phase liquide, contenant de la toluylène-diamine et du dinitrotoluène, dans laquelle le catalyseur contenant du nickel est suspendu, **caractérisé en ce que** la concentration en dinitrotoluène dans la phase liquide de la sortie de produits du réacteur est ajustée dans la zone comprise entre le réacteur et l'unité de séparation de produits en aval à une valeur dans la plage allant de 1 à 200 ppm en poids, par rapport au poids total de la phase liquide de la sortie de produits du réacteur, l'ajustement de la concentration en dinitrotoluène dans la sortie de produits liquide du réacteur dans la zone comprise entre le réacteur et l'unité de séparation de produits en aval étant réalisé en se fondant sur une mesure répétée de la concentration en dinitrotoluène dans la sortie de produits liquide du réacteur à un intervalle temporel ≤ 24 h, et la formation de produits secondaires étant réduite et la désactivation du catalyseur étant essentiellement évitée.

2. Procédé selon la revendication 1, **caractérisé en ce que** la concentration en dinitrotoluène dans la sortie de produits liquide du réacteur est ajustée à une valeur dans la plage allant de 1 à 100 ppm en poids, par rapport au poids total de la sortie de produits liquide du réacteur, de préférence à une valeur de 2 à 50 ppm en poids, par rapport au poids total de la sortie de produits liquide du réacteur, de manière davantage préférée à une valeur de 3 à 30 ppm en poids, par rapport au poids total de la sortie de produits liquide du réacteur.

3. Procédé selon la revendication 1 ou 2, **caractérisé en ce que** l'hydrogénation de dinitrotoluène en toluylène-diamine est réalisée à une température dans la plage allant de 50 à 250 °C.

4. Procédé selon l'une quelconque des revendications 1 à 3, **caractérisé en ce que** l'hydrogénation de dinitrotoluène en toluylène-diamine est réalisée à une pression dans la plage allant de 5 à 50 bar.

5. Procédé selon l'une quelconque des revendications 1 à 4, **caractérisé en ce que** la phase liquide de la sortie de produits contient de l'aminonitrotoluène et **en ce que** la concentration en aminonitrotoluène dans la phase liquide de la sortie de produits du réacteur dans la zone comprise entre le réacteur et l'unité de séparation de produits est ajustée à une valeur dans la plage allant de 0 à 2 000 ppm en poids, par rapport au poids total de la phase liquide de la sortie de produits.

6. Procédé selon l'une quelconque des revendications 1 à 5, **caractérisé en ce que** le réacteur est choisi dans le groupe constitué par un réacteur à cuve agitée, un réacteur à boucle et une colonne à bulles.

7. Procédé selon l'une quelconque des revendications 1 à 6, **caractérisé en ce que** le réacteur est un réacteur à boucle à jet comprenant un circuit extérieur et intérieur.

8. Procédé selon l'une quelconque des revendications 1 à 7, **caractérisé en ce que** l'unité de séparation de produits est choisie dans le groupe constitué par les filtres, les décanteurs statiques et les décanteurs dynamiques.

9. Procédé selon l'une quelconque des revendications 1 à 8, **caractérisé en ce que** le catalyseur suspendu contient en plus du nickel en tant que métal catalytiquement actif au moins un métal du groupe de transition I, II, V, VI et/ou VIII du tableau périodique.

10. Procédé selon l'une quelconque des revendications 1 à 9, **caractérisé en ce que** le catalyseur présente une teneur en nickel comprise entre 0,1 et 99 % en poids, par rapport au poids total du catalyseur.

11. Procédé selon l'une quelconque des revendications 1 à 10, **caractérisé en ce que** le catalyseur contient au moins un composant support oxydique.

12. Procédé selon l'une quelconque des revendications 1 à 11, **caractérisé en ce que** le catalyseur contient du platine et du nickel.

13. Procédé selon l'une quelconque des revendications 1 à 11, **caractérisé en ce que** le catalyseur contient du palladium, du nickel et du fer.

14. Procédé selon l'une quelconque des revendications 1 à 13, **caractérisé en ce que** le catalyseur est utilisé en une quantité de 0,1 à 15 % en poids, par rapport au poids total du mélange réactionnel dans le réacteur.

15. Procédé selon l'une quelconque des revendications 1 à 14, **caractérisé en ce que** la réaction est réalisée dans un mélange triphasé constitué par une phase gazeuse contenant de l'hydrogène, un catalyseur suspendu et une phase liquide, contenant 0 à 40 % en volume d'un alcool, 10 à 60 % en volume d'eau et 20 à 70 % en volume de toluylène-diamine.
